# EUROPEAN PATENT SPECIFICATION

(11) **EP 0 839 573 B1**
(45) Date of publication and mention of the grant of the patent: **18.12.2002**
(21) Application number: 97118815.6
(22) Date of filing: 29.10.1997
(51) Int. Cl.: B01J 23/00, B01J 23/50, B01J 27/12, B01J 23/66, B01J 23/68

(54) **Hydrogenation process and catalyst therefor comprising palladium and silver deposited on a spinel support**
Hydrierungsverfahren und Katalysator enthaltend Palladium und Silber auf einem spinellhaltigen Träger
Procédé et catalyseur d'hydrogénation comprenant du palladium et de l'argent déposés sur un support à base de spinelle

(30) Priority: 30.10.1996 US 740527
(43) Date of publication of application: 06.05.1998
(73) Proprietor: PHILLIPS PETROLEUM COMPANY, Bartlesville Oklahoma (US)
(72) Inventor: Cheung, Tin-Tack Peter, Bartlersville, OK 74006 (US); Sasaki, Kazuhiko James, Bartlersville, OK 74006 (US); Johnson, Marvin Merrill, Bartlersville, OK 74006 (US); Brown, Scott Hudson, Bartlersville, OK 74006 (US)
(74) Representative: Ricker, Mathias, Dr. Dipl.-Chem.

(56) References cited:
- EP-A- 0 645 171
- DE-A- 4 128 629
- US-A- 4 484 015
- US-A- 4 806 159
- US-A- 5 455 376
- US-A- 5 489 565

## Description

### FIELD OF THE INVENTION

This invention relates to a composition and a process useful for catalytically hydrogenating an unsaturated hydrocarbon compound.

### BACKGROUND OF THE INVENTION

It is well known to one skilled in the art that an unsaturated hydrocarbon compound can be produced by a thermal cracking process. For example, a fluid stream containing a saturated hydrocarbon such as, for example, ethane, propane, butane, pentane, naphtha, or combinations of any two or more thereof can be fed into a thermal (or pyrolytic) cracking furnace. Within the furnace, the saturated hydrocarbon is converted to an unsaturated hydrocarbon compound such as, for example, ethylene and propylene. Unsaturated hydrocarbons are an important class of chemicals that find a variety of industrial uses. For example, ethylene can be used as a monomer or comonomer for producing a polyolefin. Other uses of unsaturated hydrocarbons are well known to one skilled in the art.

However, an unsaturated hydrocarbon produced by a thermal cracking process generally contains an appreciable amount of less desirable alkyne(s) or diolefin(s). For example, ethylene produced by thermal cracking of ethane is generally contaminated with some acetylene which must be selectively hydrogenated to ethylene, but not to ethane, in a hydrogenation reaction.

The selective hydrogenation of alkynes is generally, commercially carried out in the presence of an alumina-supported palladium catalyst. In the case of the selective hydrogenation of acetylene to ethylene, a palladium and silver catalyst supported on an alumina can be employed. See for example U.S. Patent No. 4,404,124 and U.S. patent No. 4,484,015, . The operating temperature for this hydrogenation process is selected such that essentially all alkyne, such as, for example, acetylene is hydrogenated to its corresponding alkene such as, for example, ethylene thereby removing the alkyne from the product stream while only an insignificant amount of alkene is hydrogenated to alkane. Such a selective hydrogenation process minimizes the losses of desired unsaturated hydrocarbons and, in the front-end and total cracked gas processes, avoids a "runaway" reaction which is difficult to control, as has been pointed out in the above-identified patents.

It is generally known to those skilled in the art that impurities such as carbon monoxide, H₂S, COS, mercaptans and organic sulfides which are present in an alkyne-containing feed or product stream can poison and deactivate a palladium-containing catalyst. For example, carbon monoxide is well known to temporarily poison or inactivate such a hydrogenation catalyst thereby making the selective hydrogenation less effective.

A palladium-containing "skin" catalyst in which palladium is distributed on the surface or "skin" of the catalyst has been developed which is known to be more selective and active than a non-skin catalyst in converting acetylene in an ethylene stream to ethylene. See for example, U.S. Patent No. 4,484,015. It is well known that the catalyst selectivity is determined, in part, by the skin thickness. Generally, catalyst selectivity decreases as the skin thickness increases. There is therefore an ever-increasing need to develop a catalyst having a better "skin" on the catalyst for a better selective hydrogenation of an alkyne to an alkene.

Palladium supported on alumina has been successfully used in dry hydrogenation processes for many years. However, in some processes such as the so-called "total cracked gas" process in which the steam is not removed from the olefins stream, the selective hydrogenation of an alkyne to alkene must be accomplished in the presence of steam. In such processes, the alumina supported catalyst may have a much shorter life because alumina is not stable in steam. Therefore, there is also an increasing need to develop a palladium catalyst on a steam-stable support.

US-A-5 489 565 discloses a catalyst composition comprising palladium, silver, an alkali metal fluoride, and an inorganic support material. This catalyst composition is employed in the selective hydrogenation of C₄-C₁₀ diolefins to the corresponding monoolefins.

As such, development of an improved palladium catalyst and a process therewith in the selective hydrogenation of an alkyne to an alkene in the presence of an impurity would be a significant contribution to the art and to the economy.

### SUMMARY OF THE INVENTION

It is an object of this invention to provide a composition that can be used for selectively hydrogenating an alkyne to an alkene. It is another object of this invention to provide a palladium-containing catalyst composition wherein the palladium is better distributed on the skin of the composition, as compared to known "skin" catalysts. It is also an object of this invention to provide a process for selectively hydrogenating an alkyne to its corresponding alkene in the presence of an impurity. It is a further object of this invention to carry out a selective hydrogenation of acetylene to ethylene in the presence of an impurity. Other objects and advantages will become more apparent as this invention is more fully described hereinbelow.

According to a first embodiment of this invention, a composition as defined in claim 1 which can be used for selectively hydrogenating a highly unsaturated hydrocarbon such as, for example, an alkyne or a diolefin, is provided. The composition comprises palladium, silver, and a spinel such as a metal aluminate or a metal titanate wherein the metal of the spinel is preferably selected from the group consisting of zinc, magnesium, iron, manganese, any metal that can form a spinel structure, such as beryllium, strontium, barium, radium, tin, zirconium, molydenum, ruthenium, rhodium, cobalt, germanium, calcium, and combinations of any two or more thereof.

According to a second embodiment of this invention, a process as defined in claim 9 which can be used for selectively hydrogenating a highly unsaturated hydrocarbon to a less unsaturated hydrocarbon is provided. The process comprises contacting a highly unsaturated hydrocarbon with hydrogen, in the presence of a catalyst composition, under a condition sufficient to effect a selective hydrogenation of the highly unsaturated hydrocarbon. The catalyst composition is the same as the composition disclosed in the first embodiment of this invention.

### DETAILED DESCRIPTION OF THE INVENTION

As used in the present invention, the term "fluid" denotes gas, liquid, or combination thereof. The term "saturated hydrocarbon" is referred to as any hydrocarbon which can be converted to an unsaturated hydrocarbon such as an olefinic compound by a thermal cracking process. An "unsaturated hydrocarbon" as used in this application is a hydrocarbon having at least one double bond between carbon atoms in the molecule. Generally, examples of saturated hydrocarbons include, but are not limited to, ethane, propane, butanes, pentanes, hexanes, octanes, decanes, naphtha, and combinations of any two or more thereof. Examples of unsaturated hydrocarbons include, but are not limited to, monoolefins such as ethylene, propylene, butenes, pentenes, hexenes, octenes, and decenes; aromatic compounds such as naphthalene; alkynes such as acetylene, propyne, and butynes; diolefins such as butadienes, pentadienes (including isoprene), hexadienes, octadienes, and decadienes; and combinations of two or more thereof. The term "highly unsaturated hydrocarbon" refers to a hydrocarbon which contains a triple bond or two or more double bonds in a molecule. The term "less unsaturated hydrocarbon" refers to a hydrocarbon in which the triple bond in the high unsaturated hydrocarbon is hydrogenated to a double bond or a hydrocarbon in which the number of double bonds is one less than that in the highly unsaturated hydrocarbon. The term "selective hydrogenation" is referred to as a hydrogenation process which converts a highly unsaturated hydrocarbon such as an alkyne or a diolefin to a less unsaturated hydrocarbon such as a monoolefin without hydrogenating the less unsaturated hydrocarbon to a saturated or a more saturated hydrocarbon such as alkane.

According to the first embodiment of this invention, a composition as defined in claim 1 which can be used to selectively hydrogenate an alkyne or a diolefin to monoolefin is provided. The composition comprises, consists essentially of, or consists of palladium, silver, and a spinel such as, for example, a metal aluminate or a metal titanate wherein the palladium is present on the skin of the composition and the silver can be distributed on the skin of or throughout the composition, and the metal of the spinel is the same as that disclosed above. The presently preferred spinel is zinc aluminate, zinc titanate, magnesium aluminate, or combinations of any two or more thereof. These spinels are readily available and effective. The term "skin" is referred to as the surface of the composition. The thickness of the skin is in the range of from 1 to 100 µm, preferably 10 to 100 µm. Presently, it is preferred that palladium and silver are supported on the spinel.

Generally, palladium can be present in the composition in any weight percent (%) so long as the palladium is substantially concentrated on the skin of the composition and the weight % is effective to selectively hydrogenate an alkyne to an alkene, or a diolefin to a monoolefin. The weight % of palladium can be in the range of from 0.0001 to 3, preferably 0.0005 to 1.5, and most preferably 0.001 to 1.5 , in particular 0.001 to 1.0%. Similarly, silver can be present in the composition in any weight % as long as the weight % can effect the selective hydrogenation of an alkyne to an alkene, or a diolefin to a monoolefin. Generally, silver can be present in the composition in the range of from 0.0003 to 20, preferably 0.003 to 10, and most preferably 0.003 to 5, in particular 0.001 to 3 weight %. Optionally, the composition can also comprise, consist essentially of, or consist of palladium, silver, an alkali metal or alkali metal-containing compound, and a spinel such as a metal titanate or a metal aluminate. The alkali metal or alkali metal-containing compound can be present in the composition in any weight % that can effect the selective hydrogenation of an alkyne to an alkene, or a diolefin to a monoolefin, and in the range of from 0.001 to 10, preferably 0.005 to 5, and most preferably 0.01 to 2 weight %. The presently preferred alkali metal compound is an alkali metal fluoride such as, for example, potassium fluoride. Generally, the spinel can make up the rest of the composition.

The composition can be in any physical form so long as the physical form can be used as a catalyst for selectively hydrogenating an alkyne to an alkene, or a diolefin to a monoolefin. Generally, it is preferred the physical form be spherical or cylindrical for such form is easy to handle. The composition has a size in the range of from 0.1 to 20, preferably 0.5 to 15, and most preferably 1 to 10 mm in diameter. The composition can have a surface area of from 0.1 to 50, preferably 0.5 to 10 m²/g, as determined by the well-known BET method employing nitrogen.

Generally, any spinel can be used in the composition so long as the composition can effect the selective hydrogenation of an alkyne to an alkene, or a diolefin to a monoolefin. As disclosed above, the metal of the spinel can include magnesium, zinc, iron, manganese, any metal that can form a spinel, and combinations of any two or more thereof. Examples of suitable spinels include, but are not limited to, zinc aluminate, magnesium aluminate, zinc titanate, calcium aluminate, manganese aluminate, ferrous aluminate, calcium titanate, magnesium titanate, and combinations of any two or more thereof.

The composition can be prepared by any suitable techniques. Generally, the palladium can be placed on a spinel in any suitable manner that will yield a composition meeting the above-described parameters. The presently preferred technique involves impregnating a spinel with an aqueous solution of a suitable palladium compound. Generally, the extent of penetration of the palladium can be controlled by adjustment of the acidity of the solution with an acid such as, for example, hydrochloric acid.

Examples of suitable palladium compounds include, but are not limited to, palladium chloride, palladium bromide, palladium iodide, palladium acetate, palladium nitrate, palladium sulfate, palladium sulfide, palladium acetylacetonate, and combinations of any two or more thereof. The presently preferred palladium compound is palladium chloride for it is readily available.

One can use any suitable method to determine whether substantial weight percent of the composition particles have the palladium concentrated in an area within certain distance of the exterior surface. One technique currently favored is the electron microprobe which is well known to one skilled in the art. Another technique involves breaking open a representative sample of calcined catalyst pills and treating them with a dilute alcoholic solution of N,N-dimethyl-para-nitrosoaniline. The treating solution reacts with the oxidized palladium to give a red color which can be used to evaluate the distribution of the palladium. Still another technique involves breaking open a representative sample of calcined catalyst pills followed by treatment with a reducing agent such as, for example, hydrogen to change the color of the skin.

The silver can be distributed on the skin of or throughout the composition in any suitable and effective manner. Examples of suitable silver compounds include, but are not limited to, silver chloride, silver bromide, silver iodide, silver acetate, silver nitrate, silver sulfate, silver fluoride, silver perchlorate, and combinations of any two or more thereof. It is currently preferred to employ an aqueous silver nitrate solution in a quantity greater than that necessary to fill the pore volume of the composition. Generally, the weight ratio of silver to palladium can be in the range of from 0.1:1 to 20:1, preferably 1:1 to 10:1, and most preferably 3:1 to 8:1.

The impregnated composition can be dried at a temperature in the range of 25°C to 150°C, preferably 25°C to 120°C, and most preferably 30°C to 120°C, followed by calcining at a temperature of from 200°C to 1,200°C, preferably 275°C to 850°C, and most preferably 400°C to 700°C for 1 to 40 hours, preferably 1 to 30 hours, and most preferably 2 to 25 hours.

Any alkali metal-containing compounds can be used in the composition if the resulting composition can effect a selective hydrogenation of an alkyne to an alkene, or a diolefin to a monoolefin. Examples of suitable alkali metal compounds include sodium fluoride, potassium fluoride, lithium fluoride, rubidium fluoride, cesium fluoride, sodium iodide, potassium iodide, lithium iodide, rubidium iodide, cesium iodide, sodium chloride, potassium chloride, lithium chloride, rubidium chloride, cesium chloride, sodium bromide, potassium bromide, lithium bromide, rubidium bromide, cesium bromide, sodium hydroxide, potassium hydroxide, lithium hydroxide, rubidium hydroxide, cesium hydroxide, sodium oxide, potassium oxide, lithium oxide, rubidium oxide, cesium oxide, sodium carbonate, potassium carbonate, lithium carbonate, rubidium carbonate, cesium carbonate, sodium nitrate, potassium nitrate, lithium nitrate, rubidium nitrate, cesium nitrate, and combinations of any two or more thereof. The presently preferred alkali metal-containing compound is potassium fluoride for it is effective in the selective hydrogenation. The alkali metal-containing compound can be incorporated into a spinel by any methods known to one skilled in the art. For example, an alkali metal-containing compound can be impregnated or sprayed onto a spinel before it is impregnated with a suitable palladium compound, and preferably also with a suitable silver compound. Alternatively, the alkali metal-containing compound can be incorporated, for example, by impregnation or spraying onto the composition simultaneously with or after the impregnation with a suitable palladium compound. The alkali metal-containing compound can also be incorporated into a spinel between the palladium and silver impregnation steps or after the impregnation with palladium and silver compounds. Alternatively, one can also apply a "wet reducing" step which is a treatment with dissolved reducing agents such as hydrazine, alkali metal borohydrides, aldehydes such as formaldehyde, carboxylic acids such as formic acid or ascorbic acid, reducing sugars such as dextrose.

In the second embodiment of this invention, a process as defined in claim 9 for selectively hydrogenating a highly unsaturated hydrocarbon such as, for example, an alkyne or a diolefin, to a less unsaturated hydrocarbon such as, for example, a monoolefin is provided. The process comprises, consists essentially of, or consists of contacting a highly unsaturated hydrocarbon with hydrogen, in the presence of a catalyst under a condition sufficient to effect the selective hydrogenation of an alkyne to an alkene, or a diolefin to a monoolefin. Though any highly unsaturated hydrocarbon can be used in the process, it is presently preferred that an alkyne or diolefin containing 2 to 12, preferably 2 to 10, and most preferably 2 to 6 carbon atoms be used.

The catalyst composition is the same composition described above in the first embodiment of this invention. Hydrogen can be present either in the feed stream containing the highly unsaturated hydrocarbon or in a hydrogen-containing fluid which is mixed with the feed stream before contacting with the catalyst composition. If a hydrogen-containing fluid is used, it can be a substantially pure hydrogen or any fluid containing sufficient concentration of hydrogen to effect the hydrogenation. It can also contain other gases such as, for example, nitrogen, methane, carbon monoxide, carbon dioxide, steam, or combinations of any two or more thereof so long as the hydrogen-containing fluid contains sufficient concentration of hydrogen to effect the hydrogenation.

Optionally, the catalyst can be first treated, prior to the selective hydrogenation, with a hydrogen-containing fluid to activate the catalyst. Such reductive, or activation, treatment can be carried out at a temperature in the range of 20°C to 200°C, preferably 25°C to 150°C, and most preferably 30°C to 125°C for a time period of 1 minute to 30 hours, preferably 0.5 to 25 hours, and most preferably 1 to 20 hours. During this reductive treatment, palladium and silver compounds (primarily oxides) which may be present in the catalyst composition after the drying step and the calcining step described above are substantially reduced to palladium and silver metal. When this optional reductive treatment is not carried out, the hydrogen gas present in the reaction medium accomplishes this reduction of oxides of palladium and silver during the initial phase of the selective hydrogenation reaction of this invention.

The selective hydrogenation process of this invention is carried out by contacting a fluid which comprises the highly unsaturated hydrocarbon, in the presence of hydrogen, with the catalyst composition disclosed above. The highly unsaturated hydrocarbon can further comprise a fluid which can be water, steam, water containing a soluble or insoluble substance, or combinations of any two or more thereof. Preferably the fluid containing the highly unsaturated hydrocarbon is an unsaturated alkene stream containing an alkyne, a diolefin, or both as an impurity, generally at a level of 1 mg/Kg (ppm) to 50,000 ppm of the fluid. The highly unsaturated hydrocarbon can be, for example, an alkyne, a diolefin, or combinations of any two or more thereof. Examples of suitable alkynes include, but are not limited to, acetylene, propyne, 1-butyne, 2-butyne, 1-pentyne, 2-pentyne, 3-methyl-1-butyne, 1-hexyne, 1-heptyne, 1-octyne, 1-nonyne, 1-decyne, and combinations of any two or more thereof. The presently preferred alkyne is acetylene. These alkynes are primarily hydrogenated to the corresponding alkenes. For example, acetylene is primarily hydrogenated to ethylene, propyne is primarily hydrogenated to propylene, and the butynes are primarily hydrogenated to the corresponding butenes (1-butene, 2-butenes). Examples of suitable diolefins include, but are not limited to, butadienes, isoprene, pentadienes, cyclopentadienes, hexadienes, cyclohexadienes, octadienes, cyclooctadienes, decadienes, and combinations of any two or more thereof. These diolefins are selectively hydrogenated to their corresponding monoolefins. In order to best attain substantially complete selective hydrogenation of the highly unsaturated hydrocarbon, there should be at least about one mole of hydrogen for each mole of the highly unsaturated hydrocarbon present. A fluid containing the highly unsaturated hydrocarbon and hydrogen can be introduced into a reactor. Alternatively, a fluid containing the highly unsaturated hydrocarbon and a hydrogen-containing fluid can be introduced into a reactor separately, contemporaneously introduced, or premixed before their introduction to a reactor to contact with the catalyst which is generally laced in the reactor before introduction of the fluid(s) into the reactor. Any reactors known to one skilled in the art for selective hydrogenation can be employed in the present invention. The process of the invention can be carried out in a batch, semi-batch, or continuous mode.

The term "impurity" used herein denotes any component in a fluid stream that is not a major component. Examples of impurities other than an alkyne or a diolefin include, but are not limited to carbon monoxide, hydrogen sulfide, carbonyl sulfide (COS), mercaptans (RSH), organic sulfides (RSR), organic disulfides (RSSR), methane, ethane, propane, butane, carbon dioxide, water, alcohols, ethers, aldehydes, ketones, carboxylic acids, esters, other oxygenated compounds, and combinations of two or more thereof, wherein each R can be an alkyl or cycloalkyl or aryl group containing 1 to 15, preferably 1 to 10 carbon atoms. Generally, each impurity is present in the fluid stream in trace amounts. For example, an impurity can be present at a level of less than 1 weight percent (%).

The temperature necessary for the selective hydrogenation of the highly unsaturated hydrocarbon such as, for example, an alkyne, to a less unsaturated hydrocarbon such as, for example, an alkene is any temperature that can effect the conversion of, for example, an alkyne to an alkene. It generally depends largely upon the activity and selectivity of a catalyst, the amounts of impurities in the fluid, and the desired extent of removal of impurities. Generally, a reaction temperature can be in the range of 10°C to 300°C, preferably 20 to 250°C, and most preferably 30 to 200°C. Any suitable reaction pressure can be employed. Generally, the total pressure is in the range of 0.345 to 10.35 (50 to 1,500), preferably 0.518 to 8.28 (75 to 1,200), and most preferably 0.69 to 69 MPa gauge (100 to 1,000 pounds per square inch gauge (psig)). The liquid or gas hourly space velocity of the fluid feed can also vary over a wide range. Typically, the gas space velocity can be in the range of 10 to 20,000 m³ of the fluid per m³ of catalyst per hour, more preferably 50 to 12,500 m³/m³/hour, and most preferably 100 to 8,000 m³/m³/hour. The liquid space velocity of the feed can be in the range of from 0.001 to 200, preferably 0.01 to 100, and most preferably 0.1 to 50 m³/m³/hour. The molar ratio of hydrogen to the highly unsaturated hydrocarbon is in the range of 0.5:1 to 10,000:1, preferably 1:1 to 5,000:1 and most preferably 1:1 to 1,000:1. The hourly space velocity of the hydrogen-containing fluid, if separately fed to a reactor containing a selective hydrogenation catalyst, is chosen so as to provide a molar ratio of H₂ to a highly unsaturated hydrocarbon in the range of 0.5:1 to 10,000:1, preferably 1:1 to 5,000:1 and most preferably 1:1 to 1,000:1.

Regeneration of the catalyst composition can be accomplished by heating the catalyst composition in air (at a temperature which preferably does not exceed 700°C) so as to burn off any impurities such as, for example, organic matter and/or char that may have accumulated on the catalyst composition. Optionally, the oxidatively regenerated composition is reduced with H₂ or a suitable hydrocarbon (as has been described above) before its reemployment in the selective hydrogenation of this invention.

The following examples are presented to further illustrate this invention and are not to be construed as unduly limiting its scope.

### EXAMPLE I

This comparative example illustrates that a catalyst having a better "skin" of palladium is produced by using a spinel.

The impregnating solution was prepared by dissolving 0.018 gram of PdCl₂ into a small vial (about 10 ml) with 4 drops of concentrated HCl. The content was heated to almost dryness while the vial was being swirled. Distilled water (5 grams) was added to the vial to mix and dissolve the palladium salt to prepare a PdCl₂ solution. An aliquot (0.94 gram) of the thus-prepared PdCl₂ solution was then added to an α-alumina support (1.025 g; about 3-7 m²/g surface area and 0.22-0.32 cm³/g; in 4.76 mm (3/16 inch) tablet form) which was obtained from UCI (United Catalysts Inc., Louisville, Kentucky) to form a mixture in a similar vial. The alumina support was soaked in the PdCl₂ solution for about 30 minutes at 25°C while being swirled. Thereafter the excess liquid was decanted and the alumina having palladium supported thereon was placed in a porcelain dish about 76.2 mm (3 inches) in diameter, dried at 125°C for 2 hours, and then calcined at 538°C in a force draft furnace for 2 hours. This was a Pd/Al₂O₃ skin catalyst.

In a separate run, 1.07 gram of the above PdCl₂ solution was mixed with 1.159 gram of zinc aluminate (in 3.175 mm (1/8 in) tablet form; 7.3 m²/g surface area) which was obtained from Calsicat Catalyst Division of Mallinckrodt Specialty Chemicals Company, Erie, Pennsylvania. The mixture was then treated exactly as described above to produce a Pd/ZnAl₂O₄ skin catalyst.

Both skin catalysts were made in tablet forms and pills of the tablets were reduced with a hydrogen flow at about 65.6°C (150°F) until the color of the pills changed to gray-black. In the meantime, the supports were still snow-white in color. The change to gray-black color was due to the reduction of palladium oxide so that the palladium skin can be observed. Thereafter, individual tablets were split in half and the palladium skin was visually observed. It was found that the skin on zinc aluminate was much better than the skin on alumina. The term "better" used herein refers to more concentrated on the skin and less penetration into the support.

### EXAMPLE II

This example illustrates the production of a "skin" Pd/Ag/ZnAl₂O₄ catalyst.

Palladium chloride (PdCl₂, 0.0229 gram) was dissolved in 5 g of H₂O with 10 drops of concentrated HCl to form a solution in a beaker. The solution was heated to almost dryness on a hot plate. Water (9.61 g) was then added to reconstitute and prepare a PdCl₂ solution.

Zinc aluminate (58.63 grams, as 3.175 mm (1/8 inch) extrude) obtained from Calsicat which was similar to that disclosed in Example I was impregnated with the entire content of the reconstituted PdCl₂ solution. Following drying at 71.1°C (160°F) for 16 hours, the catalyst was reduced in wet H₂ (hydrogen gas was saturated with water at 25°C) for 16 hours at 380°C and then cooled to 160°C followed by a purge of nitrogen and air for about 4 hours. The dried catalyst was then calcined in air at 380°C for 5 hours.

A solution containing 0.1724 gram of AgNO₃ in 33.24 grams of water was used to soak the calcined catalyst for about 30 minutes and then dried at 82.2°C (180°F) for 16 hours to prepare a dried, Ag-impregnated catalyst. The dried, Ag-impregnated catalyst was calcined at 200°C for 16 hours in air and then 4 hours at 370°C. The catalyst was a Pd/Ag/ZnAl₂O₄ skin catalyst.

Twenty milliliters of the extrudates catalyst were packed into a water jacketed stainless steel reactor 12.7 mm (½ inch) inner diameter; 457.2 mm (18 inches) long). Thermocouples were inserted into a thermal well which ran through the center and coaxial with the reactor which was heated with an external water bath. The hydrocarbon feed used is shown in Table I. After the catalyst was treated with H₂ at 1.38 MPa gauge (200 psig) (90 cm³/min) at room temperature (25°C) for 16 hours, the feed at 1.38 MPa gauge (200 psig) was introduced into the reactor at 38.9°C (102°F). The feed rate was about 900 cm³/minute. At intervals, the reactor effluent was sampled and analyzed with a gas chromatograph. The results of the test are shown in Table I.

**Table Ia**

| T((°F)°C) | C₂⁼/C₂ | Acetylene | C₄'s | C₆'s |
|---|---|---|---|---|
| (102) 38.9 | 3.692 | 0 | 0.015 | t |
| (96) 35.6 | --- | 0.008 | 0.012 | 0.002 |
| (99) 37.2 | 3.709 | 0.004 | 0.016 | 0.005 |
| (103) 39.4 | 3.695 | 0 | 0.015 | 0.005 |
| (122) 50.0 | 3.569 | 0 | 0.016 | 0.008 |
| (138) 58.9 | 3.349 | 0 | 0.015 | 0.007 |
| (161) 71.7 | 2.803 | 0 | 0.012 | 0.005 |
| ^{a}The feed contained 10.84 mole % ethane, 40.39 mole % ethylene, 0.238 mole % acetylene, about 0.02 mole % CO, 34.698 mole % methane, and essentially no ethers. The values shown are mole % except that C₂ ⁼ /C₂ is molar ratio. A zero (0) mole % acetylene denotes acetylene content was less than 10 parts per million by moles. The C₂⁼ run away condition was when the effluent C₂ was at 12.84 mole % while the C₂⁼ in the effluent decreased to 38.39%. | | | | |

### EXAMPLE III

This comparative example illustrates a selective hydrogenation with a commercially available Pd/Ag/Al₂O₃ catalyst.

The catalyst was a commercial Pd/Ag/Al₂O₃ catalyst which contained 0.018 weight % Pd, 0.065 weight % Ag and about 99 weight % alumina. It had a BET/N₂ surface area of 3 to 5 m²/g, and had been prepared substantially in accordance with the method described in U.S. Patent 4,404,124 (column 4, lines 32-45), disclosure of which is herein incorporated by reference. This catalyst was provided by United Catalysts Inc. (UCI), Louisville, KY.

The catalyst was used in a selective hydrogenation test which was carried out the same as that disclosed in Example II. The results are shown in Table II.

**Table IIa**

| T((°F)°C) | C₂⁼/C₂ | Acetylene | C₄'s | C₆'s |
|---|---|---|---|---|
| (101) 38.3 | --- | 0.093 | 0.011 | 0.006 |
| (108) 42.2 | --- | 0.050 | 0.016 | 0.008 |
| (117) 47.2 | 3.696 | 0.002 | 0.017 | 0.008 |
| (119) 48.3 | 3.682 | 0 | 0.019 | 0.008 |
| (138) 58.9 | 3.436 | 0 | 0.019 | 0.007 |
| (151) 66.1 | 3.163 | 0 | 0.017 | 0.006 |
| (160) 71.1 | 2.887 | 0 | 0.015 | 0.005 |
| ^{a}See Table I footnote a. | | | | |

### EXAMPLE IV

This example further illustrates the use of a Pd/Ag/ZnAl₂O₄ skin catalyst in a selective hydrogenation of acetylene to ethylene.

The catalyst was made with 30 grams of zinc aluminate, 30 grams of PdCl₂ solution, and 30 grams of AgNO₃ solution. The PdCl₂ solution was prepared by diluting 12 grams of 0.1 g Pd/100 g solution of PdCl₂ to 60 grams with water and the AgNO₃ solution was prepared by dissolving 0.2268 grams of AgNO₃ in 60 grams of water.

An aliquot of PdCl₂ solution (30 grams) was added to zinc aluminate in a ceramic bowl to form a mixture. After 1 hour at 23 °C, the solution was drained, the solid was dried at 125°C for 30 minutes, and then calcined for 2 hours at 454°C. Thirty grams of AgNO₃ solution was then added to the calcined catalyst to form a second mixture. After 1 hour at 23 °C, the solution was drained, the solid was dried at 125°C for 30 minutes, and then calcined at 454°C for 2 hours to prepare a Pd/Ag/ZnAl₂O₄ skin catalyst containing 0.02 weight % Pd, 0.12 weight % Ag, and 99.86 weight % ZnAl₂O₄.

The selective hydrogenation was carried out the same as that disclosed in Example II except that the catalyst made in this Example was used. The results are shown in Table III.

In a separate, comparative run, a catalyst was prepared by the same procedure except that an α-alumina was used in place of zinc aluminate. The thus-obtained Pd/Ag/Al₂O₃ skin catalyst was used to selectively hydrogenate acetylene as disclosed in Example II. The results are shown in Table IV.

**Table IIIa**

| Temp (°C(°F)) | Ethane | Ethylene | Acetylene | C₄⁼'s |
|---|---|---|---|---|
| feed: | 0 | 67.995 | 3480 | 0 |
| 37.2(99) | 0 | 68.249 | 663 | 417 |
| 40.0(104) | 795 | 68.077 | 134 | 514 |
| 40.6(105) | 1014 | 68.026 | 83 | 527 |
| 41.1(106) | 961 | 68.139 | 81 | 514 |
| 42.2(108) | 1276 | 68.043 | 0 | 360 |
| 42.8 (109) | 1806 | 68.021 | 0 | 206 |
| 43.3 (110) | 1623 | 68.021 | 0 | 509 |
| 45.0 (113) | 2164 | 67.970 | 0 | 502 |
| 45.6 (114) | 2287 | 67.964 | 0 | 350 |
| 49.4 (121) | 3962 | 67.859 | 0 | 469 |
| 54.4 (130) | 6894 | 67.547 | 0 | 441 |
| 59.4 (139) | 11475 | 67.056 | 0 | 407 |
| 62.8 (145) | 14943 | 66.734 | 0 | 368 |
| 67.2 (153) | 21135 | 66.150 | 0 | 345 |
| 71.1 (160) | 29370 | 65.304 | 0 | 322 |
| ^{a}C₂ ⁼ runaway target wt %=64.995 (feed - 3%). The values shown for ethylene are weight %. All other are ppm by weight. The feed was a de-ethanized ethylene feed containing 300 ppm CO and there was little or no heavies (>C₄) in the product stream. | | | | |

The results in Table III show that a skin catalyst prepared on zinc aluminate spinel as support had very good catalyst activity because it had a low cleanup temperature of 41.7°C (107°F). The term "cleanup temperature" is defined as the temperature at which the acetylene concentration at the reactor exit is less than 20 ppm by weight. The results in Table III also show that the Pd/Ag/ZnAl₂O₄ skin catalyst had a high run away temperature. The term "runaway temperature" is defined as the temperature above which uncontrollable hydrogenation of ethylene begins. The cleanup temperature is a measure of the catalyst activity; the lower the clean-up temperature is, the more active the catalyst is. The difference between the runaway temperature and the cleanup temperature is a measure of the selectivity of the catalyst; a more selective catalyst has a larger difference.

**Table IVa**

| Temp (°C(°F)) | Ethane | Ethylene | Acetylene | C₄⁼'s |
|---|---|---|---|---|
| feed: | 0 | 67.963 | 3372 | 0 |
| 41.1(106) | 910 | 68.047 | 289 | 356 |
| 43.9 (111) | 1718 | 68.110 | 106 | 495 |
| 45.0 (113) | 2001 | 67.963 | 61 | 237 |
| 45.6 (114) | 2098 | 67.921 | 49 | 298 |
| 47.2 (117) | 2249 | 67.924 | 56 | 338 |
| 47.8 (118) | 4008 | 67.746 | 0 | 333 |
| 48.3 (119) | 2856 | 67.842 | 23 | 330 |
| 53.9 (129) | 8354 | 67.344 | 0 | 261 |
| 62.8 (145) | 20401 | 66.128 | 0 | 225 |
| 63.3 (146) | 20448 | 66.258 | 0 | 214 |
| 67.2 (153) | 21855 | 66.050 | 0 | 189 |
| 71.1 (160) | 40803 | 64.207 | 0 | 186 |
| ^{a}See footnote a in Table III except that the C₂⁼ runaway target was 64.963 %. | | | | |

The results in Tables III and IV show that the invention catalyst Pd/Ag/ZnAl₂O₄ had better activity and selectivity as a comparative Pd/Ag/Al₂O₃ catalyst.

### EXAMPLE V

This example shows a Pd/Ag/ZnTiO₃ skin catalyst and the use thereof in a selective hydrogenation of acetylene to ethylene.

The zinc titanate supported skin catalyst was prepared by the same procedure described in Example IV except that zinc titanate was used as support. The hydrogenation was carried out the same as that described in Example II. The results are shown in Table V below.

**Table Va**

| Temp (°C(°F)) | Ethane | Ethylene | Acetylene | C₄⁼'s |
|---|---|---|---|---|
| feed: | 0 | 56.188 | 3035 | 174 |
| 41.1 (106) | 0 | 56.281 | 1163 | 401 |
| 44.4 (112) | 328 | 56.117 | 481 | 297 |
| 56.1 (133) | 1620 | 55.908 | 79 | 203 |
| 58.3 (137) | 1874 | 55.860 | 62 | 205 |
| 61.7 (143) | 2633 | 55.763 | 25 | 177 |
| 63.3 (146) | 12290 | 54.916 | 0 | 199 |
| 65.6 (150) | 3806 | 55.700 | 0 | 192 |
| 68.9 (156) | 20044 | 54.151 | 0 | 131 |
| 68.9 (156) | 5945 | 55.590 | 0 | 207 |
| 71.7 (161) | 6784 | 53.449 | 0 | 200 |
| 76.7 (170) | 9942 | 52.154 | 0 | 185 |
| 93.3 (200) | 45857 | 51.662 | 0 | 117 |
| ^{a}See footnote in Table III. However, the feed was a de-propanized ethylene stream containing 821 ppm CO. | | | | |

Table V also shows that the skin catalyst Pd/Ag/ZnTiO₃ had a very good catalyst activity, i.e., low clean-up temperature, and good selectivity, i.e., large difference between the clean-up temperature and run-away temperature.

### EXAMPLE VI

This example demonstrates that a magnesium aluminate can be used to prepare a skin catalyst useful for selective hydrogenation of acetylene.

Thirty grams of Halder-Topsoe CAM-9L MgAl₂O₄ tablets (about 5.1 x 5.4 mm, obtained from Halder-Topsoe, Houston, Texas, having a N₂ BET surface area of 22 m²/g and a total pore volume of 0.21 cm³/g) was cut in half with a razor blade and washed with bottled H₂O 3 times and then dried at 85 °C for 16 hours. A portion of the magnesium aluminate (24.943 grams) was covered with 24.943 grams of 0.02 weight % Pd of a PdCl₂ solution for 15 minutes with swirling and stirring. Excess solution was then poured off and blot-dried lightly followed by drying at 85°C for 3 hours.

The PdCl₂ solution was prepared as follows. First, 0.100 gram of PdCl₂ was weighed into a 30 ml beaker. Fifty drops of concentrated HCl solution was then added to the beaker. The content in the beaker was heated slowly, gently on a hot plate with swirling until almost dry. Distilled H₂O (15 ml) was added to the beaker and the content was again heated with swirling to get all solid dissolved. The resulting solution was transferred to a tared bottle and filtered through piece of Kimwipe paper towel followed by rinsing the beaker into bottle 3 to 4 times. Water was added to the bottle to make 300 grams of solution.

The dried mixture was calcined for 2 hours at 454°C. Thereafter, the calcined Pd/MgAl₂O₄ was soaked in an AgNO₃ solution, which was prepared by dissolving 0.096 grams of AgNO₃ in 24.0 grams of bottled H₂O, for 1 hour with stirring every 15 minutes. Excess AgNO₃ solution was poured off. The solid was then blot dried on paper towels, dried at 85°C for 16 hours and then calcined at 454°C in air for 2 hours.

The Pd/Ag/MgAl₂O₄ catalyst was then used to selectively hydrogenate acetylene as described in Example II. The results are shown in Table VI.

**Table VIa**

| Temp (°C(°F)) | Ethane | Ethylene | Acetylene | C₄⁼'s |
|---|---|---|---|---|
| feed: | 152 | 71.400 | 3877 | 2 |
| 37.8(100) | 775 | 71.770 | 793 | 357 |
| 41.7(107) | 609 | 71.900 | 522 | 415 |
| 46.7(116) | 1336 | 71.840 | 45 | 430 |
| 48.9 (120) | 1670 | 71.770 | 20 | 428 |
| 51.1 (124) | 2020 | 71.740 | 6 | 423 |
| 58.9 (138) | 4877 | 71.480 | 0 | 400 |
| 65.0 (149) | 8821 | 71.140 | 0 | 391 |
| 66.7 (152) | 10385 | 70.950 | 0 | 370 |
| 72.2 (162) | 17116 | 70.250 | 0 | 347 |
| 80.6 (177) | 35890 | 68.360 | 0 | 315 |
| ^{a}See footnote a in Table III except the runaway target was 68.400%. | | | | |

Table VI demonstrates that a skin catalyst supported on magnesium-aluminate also had good activity and selectivity.

The results shown in Examples II-VI are summarized in the following Table VII.

**Table VIIa**

| Catalyst | T₁ (°C(°F)) | T₂ (°C(°F)) | ΔT (°C(°F)) |
|---|---|---|---|
| Pd/Ag/Al₂O₃^{b} | 47.8 (118) | 69.4 (157) | 21.6 (39) |
| Pd/Ag/ZnAl₂O₄^{c} | 38.3 (101) | 68.3 (155) | 30.0 (54) |
| Pd/Ag/Al₂O₃^{d} | 47.2 (117) | 68.9 (156) | 21.7 (39) |
| Pd/Ag/ZnAl₂O₄^{e} | 42.2 (108) | 72.8 (163) | 30.6 (55) |
| Pd/Ag/ZnTiO₃^{f} | 62.2 (144) | 88.9 (192) | 26.7 (48) |
| Pd/Ag/MgAl₂O₄^{g} | 48.9 (120) | 80.6 (177) | 31.7 (57) |

| | | | |
|---|---|---|---|
| ^{a}T₁ denotes cleanup temperature, T₂ refers to runaway temperature, and ΔT is the difference between T₂ and T₁. The larger the ΔT is, the better the selectivity is. All data were obtained from runs with de-ethanized ethylene feed containing 300 ppm CO, except Pd/Al/ZnTiO₃, footnote f. ^{b}See Table II. Comparative Example. | | | |
| ^{c}See Table I. | | | |
| ^{d}See Table IV. Comparative Example. | | | |
| ^{e}See Table III. | | | |
| ^{f}See Table V. The run was carried out with a de-propanized ethylene feed containing 821 ppm CO. | | | |
| ^{g}See Table VI. | | | |

The results in Table VII indicate that the invention catalysts using a spinel as support are as good as, or better than, the catalysts using an alumina as support.

### EXAMPLE VII

This example illustrates the measurement of skin thickness of palladium, or the depth of palladium deposition on a support surface.

The runs were carried out as follows. For each sample, representative pellets were embedded in epoxy (obtained from Buehler, Ltd., Lake Bluff, Illinois) and allowed to polymerize overnight. A thin cut was made with a Buehler Isomet diamond saw to expose the interior of the embedded pellets. This exposed surface was polished through successively smaller grit polishing media, finishing with submicron colloidal silica. Each polished sample was coated with a thin layer of carbon in a vacuum evaporator to provide conductivity for examination in the electron microprobe.

The instrument used for the analyses was a JEOL 733 electron microprobe with Noran Voyager stage and spectrometer automation. Instrument parameters include 20 kilovolts accelerating voltage, 40 nanoamp beam current, and a focused electron beam. Line traverses to determine the metal deposition in the pellet interior were set up to start at the outer rim and to end at or near the center. Intervals between the analysis points were varied to define the deposition profile. Within the outer 200 micrometers of the traverse the interval ranged from 2 to 10 micrometers. From there to the pellet center the interval was approximately 120 micrometers. For examples that did not have silver addition, traverses to the pellet center were done on only one pellet and only the outer 200 micrometers were analyzed on the remaining pellets in order to save analysis time.

To determine the maximum depth of effective palladium deposition, the concentration at each analysis point was normalized to the maximum concentration in that traverse. For comparison purposes, the penetration depth for palladium was considered to be the point where the deposition dropped to a normalized value of 0.1 compared to 1.0 for the maximum concentration. This distance was typically at the point where the rapid decrease in the palladium deposition ended and was always before the concentration dropped below the detection limit of the analysis technique (0.02 weight %). To get finer detail from the data, the distance that corresponded to the normalized value of 0.1 was interpolated from adjacent analysis points.

The results in table VIII below indicate that palladium deposition on the alumina support was deeper into the pellet (69 micrometers) compared to 24 micrometers within zinc aluminate, and 33 micrometers within magnesium aluminate. The average depth of palladium deposition on zinc aluminate support was, in fact, statistically the same as the magnesium aluminate support.

**Table VIII**

| Catalyst Support | Alumina | Zinc Aluminate | Magnesium Aluminate |
|---|---|---|---|
| Skin thickness (µm) | 69^{a} | 24^{b} | 33 (35)^{c} |

| | | | |
|---|---|---|---|
| ^{a}The value was average of measurements of 6 catalyst pellets. The catalyst is disclosed in Example III (Comparative). | | | |
| ^{b}The value was average of measurements of 6 catalyst pellets. The catalyst is disclosed in Example II. | | | |
| ^{c}The value was average of measurements of 3 catalyst pellets. The value in the parenthesis was the result of a separate run and was average of measurements of 7 pellets in which silver was not present. The catalyst is disclosed in Example VI. | | | |

### EXAMPLE VIII

This example illustrates the superior selectivity of a skin catalyst over a non-skin catalyst.

The skin catalyst used was the same as that disclosed in Example VI. The non-skin catalyst was prepared as follows. First, a PdCl₂ solution was made by weighing 0.159 g PdCl₂ into a 10 ml beaker followed by adding 1.0 g conc. HCl to the beaker. Upon heating for 30 minutes, not to dryness, the PdCl₂/HCl liquid was transferred to a bottle and diluted therein to 100 g with bottled water.

Secondly, 25.87 g of magnesium aluminate, prepared as described in Example VI was prepared. A portion (7.998 g) of the PdCl₂ solution prepared above in a 10 ml beaker was added 32 drops of conc. HCI (1.059 g) to prepare an acidified PdCl₂. The magnesium aluminate was incipiently wetted with 7.187 g of the acidified PdCl₂ followed by drying at 85°C for 2 hours, at 100°C for 4 hours, and then 454°C for 2 hours in 200 cm³/min air to prepare Pd/MgAl₂O₄.

Thirdly, an AgNO₃ solution, prepared by dissolving 0.100 g of AgNO₃ with 24.89 g of bottled water, was poured over the Pd/MgAl₂O₄. After 1 hour, with stirring every 15 minutes, excess AgNO₃ solution was poured off and the solid was blot-dried on paper towel, further dried at 85°C for 15 hours, and calcined for 2 hours at 100°C and then 2 hours at 454°C.

The skin catalyst and non-skin catalyst, both supported on MgAl₂O₄, were tested for selective hydrogenation as described in Example II. The results are summarized in Table IX below.

**Table IXa**

| Catalyst | Pd penetration (µm) | T₁ (°C(°F)) | T₂ (°C(°F)) | Δ T(°C(°F)) |
|---|---|---|---|---|
| Skin | 33 (35)^{b} | 48.9 (120) | 80.6 (177) | 31.7 (57) |
| Non-skin | 2500^{c} | 105.0 (221) | 126.1 (259) | 21.1 (38) |

| | | | | |
|---|---|---|---|---|
| ^{a}See footnote a, Table VII. ^{b}See Table VIII. | | | | |
| ^{c}Palladium was distributed throughout the catalyst pill having a radius of about 2500 µm. | | | | |

The results in Table IX shows that a skin catalyst had much lower cleanup temperature (T₁) and higher selectivity (ΔT) than a non-skin catalyst.

The results shown in the above examples also clearly demonstrate that the present invention is well adapted to carry out the objects and attain the ends and advantages mentioned.

## Claims

1. A composition comprising palladium, silver, and a spinel wherein said palladium and silver are each present in a sufficient amount to effect hydrogenation of an unsaturated hydrocarbon having at least one double bond between carbon atoms in the molecule, wherein said palladium is present as skin distributed on the surface of said spinel, the thickness of said skin is in the range of from 1 to 100 µm, and the size of the composition is in the range of from 0.1 to 20 mm in diameter.

2. The composition of claim 1 wherein said composition further comprises an alkali metal-containing compound.

3. The composition of claim 2 wherein said alkali metal-containing compound is an alkali metal fluoride.

4. The composition of any of claims 1 to 3 wherein the metal of said spinel is selected from zinc, magnesium, calcium, beryllium, strontium. barium, radium, iron, manganese, zirconium, molybdenum, ruthenium, rhodium, cobalt, germanium, tin, and combinations of any two or more thereof, in particular wherein said spinel is selected from zinc aluminate, magnesium aluminate, zinc titanate, and combinations of any two or more thereof.

5. The composition of any of claims 2 to 4 wherein the thickness of said skin is in the range of from 10 to 100 µm.

6. The composition of any of the preceding claims wherein the weight % of said palladium is in the range of from 0.0001 to 3 %, preferably from 0.0005 to 1.5 %, most preferably from 0.001 to 1.5 %, in particular from 0.001 to 1.0 %.

7. The composition of any of the preceding claims wherein the weight % of said silver is in the range of from 0.001 to 3 %.

8. The composition of any of the preceding claims wherein the weight ratio of silver to palladium is in the range of from 0.1 : 1 to 20 : 1, preferably from 3 : 1 to 8 : 1.

9. A process comprising contacting a highly unsaturated hydrocarbon containing a triple bond or two or more double bonds in a molecule, in the presence of hydrogen, with a composition under a condition sufficient to effect selective hydrogenation of said highly unsaturated hydrocarbon to a less unsaturated hydrocarbon wherein said composition is as defined in any of the preceding claims.

10. The process of claim 9 wherein said hydrogen is present in said highly unsaturated hydrocarbon.

11. The process of claim 9 wherein said hydrogen is fed separately and mixed with said highly unsaturated hydrocarbon prior to said contacting with said composition.

12. The process of any of claims 9 to 11 wherein said highly unsaturated hydrocarbon comprises a fluid selected from the group consisting of water, steam, water containing a soluble or insoluble substance, and combinations of any two or more thereof.

## Patentansprüche

1. Zusammensetzung, umfassend Palladium, Silber und einen Spinell, wobei das Palladium und Silber beide in einer ausreichenden Menge vorhanden sind, um eine Hydrierung eines ungesättigten Kohlenwasserstoffs mit mindestens einer Doppelbindung zwischen Kohlenstoffatomen im Molekül, zu bewirken, wobei das Palladium als Außenschicht bzw. Haut auf der Oberfläche des Spinells verteilt ist, die Dikke dieser Außenschicht im Bereich von 1 bis 100 µm und die Größe des Durchmessers der Zusammensetzung im Bereich von 0,1 bis 20 mm liegt.

2. Zusammensetzung nach Anspruch 1, wobei die Zusammensetzung weiterhin eine ein Alkalimetall enthaltende Verbindung umfasst.

3. Zusammensetzung nach Anspruch 2, wobei die Alkalimetall enthaltende Verbindung ein Alkalimetallfluorid ist.

4. Zusammensetzung nach einem der Ansprüche 1 bis 3, wobei das Metall des Spinells ausgewählt ist aus Zink, Magnesium, Calcium, Beryllium, Strontium, Barium, Radium, Eisen, Mangan, Zirkonium, Molybdän, Ruthenium, Rhodium, Kobalt, Germanium, Zinn und Kombinationen von beliebigen zwei oder mehreren davon, insbesondere, wobei der Spinell ausgewählt ist aus Zinkaluminat, Magnesiumaluminat, Zinktitanat und Kombinationen von beliebigen zwei oder mehreren davon.

5. Zusammensetzung nach einem der Ansprüche 2 bis 4, wobei die Dicke der Außenschicht im Bereich von 10 bis 100 µm liegt.

6. Zusammensetzung nach einem der vorhergehenden Ansprüche, wobei die Gewichtsprozent des Palladiums im Bereich von 0,0001 bis 3 %, vorzugsweise von 0,0005 bis 1,5 %, am meisten bevorzugt von 0,001 bis 1,5 % und insbesondere von 0,001 bis 1,0 % liegen.

7. Zusammensetzung nach einem der vorhergehenden Ansprüche, wobei die Gewichtsprozent des Silbers im Bereich von 0,001 bis 3 % liegen.

8. Zusammensetzung nach einem der vorhergehenden Ansprüche, wobei das Gewichtsverhältnis von Silber zu Palladium im Bereich von 0,1: 1 bis 20:1, vorzugsweise von 3:1 bis 8:1 liegt.

9. Verfahren, umfassend das In-Kontakt-Bringen eines hoch ungesättigten Kohlenwasserstoffs, der im Molekül eine Dreifachbindung oder zwei oder mehrere Doppelbindungen enthält, in Gegenwart von Wasserstoff mit einer Zusammensetzung unter einer Bedingung, die ausreicht, um eine selektive Hydrierung des hoch ungesättigten Kohlenwasserstoffs zu einem weniger ungesättigten Kohlenwasserstoff zu bewirken, wobei die Zusammensetzung eine Zusammensetzung ist, wie sie in einem der vorhergehenden Ansprüche definiert ist.

10. Verfahren nach Anspruch 9, wobei der Wasserstoff in dem hoch ungesättigten Kohlenwasserstoff vorhanden ist.

11. Verfahren nach Anspruch 9, wobei der Wasserstoff vor dem In-Kontakt-Bringen mit der Zusammensetzung getrennt zugeführt wird und mit dem hoch ungesättigten Kohlenwasserstoff vermischt wird.

12. Verfahren nach einem der Ansprüche 9 bis 11, wobei der hoch ungesättigte Kohlenwasserstoff ein Fluid umfasst, das ausgewählt ist aus der Gruppe bestehend aus Wasser, Dampf, Wasser, das eine lösliche oder unlösliche Substanz enthält, und Kombinationen von beliebigen zwei oder mehreren Stoffen davon.

## Revendications

1. Une composition comprenant du palladium, de l'argent et un spinelle, dans laquelle ledit palladium et ledit argent sont chacun présents selon une quantité suffisante pour réaliser l'hydrogénation d'un hydrocarbure insaturé ayant au moins une double liaison entre des atomes de carbone dans la molécule, dans laquelle ledit palladium est présent sous la forme d'une peau répartie sur la surface dudit spinelle, l'épaisseur de ladite peau se situant dans la gamme de 1 à 100 µm et la dimension de la composition se situant dans la gamme de 0,1 à 20 mm de diamètre.

2. La composition selon la revendication 1, dans laquelle ladite composition comprend, en outre, un composé renfermant un métal alcalin.

3. La composition selon la revendication 2, dans laquelle ledit composé renfermant un métal alcalin est un fluorure de métal alcalin.

4. La composition selon l'une quelconque des revendications 1 à 3, dans laquelle le métal dudit spinelle est choisi parmi le zinc, le magnésium, le calcium, le béryllium, le strontium, le baryum, le radium, le fer, le manganèse, le zirconium, le molybdène, le ruthénium, le rhodium, le cobalt, le germanium, l'étain et des combinaisons de n'importe lesquels de deux ou plusieurs de ceux-ci, en particulier dans laquelle ledit spinelle est choisi parmi l'aluminate de zinc, l'aluminate de magnésium, le titanate de zinc et des combinaisons de n'importe lesquels de deux ou plusieurs de ceux-ci.

5. La composition selon l'une quelconque des revendications 2 à 4, dans laquelle l'épaisseur de ladite peau se situe dans la gamme de 10 à 100 µm.

6. La composition selon l'une quelconque des revendications précédentes, dans laquelle le % en poids dudit palladium se situe dans la gamme de 0,0001 à 3 %, de préférence de 0,0005 à 1,5 %, mieux encore de 0,001 à 1,5 %, en particulier de 0,001 à 1,0 %.

7. La composition selon l'une quelconque des revendications précédentes, dans laquelle le % en poids dudit argent se situe dans la gamme de 0,001 à 3 %.

8. La composition selon l'une quelconque des revendications précédentes, dans laquelle le rapport pondéral de l'argent au palladium se situe dans la gamme de 0,1 : 1 à 20 : 1, de préférence de 3 : 1 à 8 : 1.

9. Un procédé comprenant la mise en contact d'un hydrocarbure très insaturé renfermant une triple liaison ou deux ou plusieurs doubles liaisons dans une molécule en présence d'hydrogène, avec une composition sous une condition suffisante pour réaliser l'hydrogénation sélective dudit hydrocarbure très insaturé en un hydrocarbure moins insaturé dans lequel ladite composition est comme défini dans l'une quelconque des revendications précédentes.

10. Le procédé selon la revendication 9, dans lequel ledit hydrogène est présent dans ledit hydrocarbure très insaturé.

11. Le procédé selon la revendication 9, dans lequel ledit hydrogène est amené séparément et mélangé avec ledit hydrocarbure très insaturé avant ladite mise en contact avec ladite composition.

12. Le procédé selon l'une quelconque des revendications 9 à 11, dans lequel ledit hydrocarbure très insaturé comprend un fluide choisi parmi le groupe comprenant l'eau, la vapeur d'eau, l'eau renfermant une substance soluble ou insoluble et des combinaisons quelconques de deux ou plusieurs de ceux-ci.
